# EUROPÄISCHE PATENTSCHRIFT

(11) **EP 2 279 024 B1**
(45) Veröffentlichungstag und Bekanntmachung des Hinweises auf die Patenterteilung: **19.10.2011**
(21) Anmeldenummer: 09731271.4
(22) Anmeldetag: 08.04.2009
(51) Int. Cl.: A61M 25/06, A61B 5/15, A61B 17/34

(54) **SPINALKANÜLE MIT LIQUOR-ERKENNUNG**
SPINAL CANNULA HAVING LIQUOR DETECTION
CANULE SPINALE À DÉTECTION DE LIQUIDE CÉPHALORACHIDIEN

(30) Priorität: 08.04.2008 DE 102008017807
(43) Veröffentlichungstag der Anmeldung: 02.02.2011
(73) Patentinhaber: B. Braun Melsungen AG, 34212 Melsungen (DE)
(72) Erfinder: KATERKAMP, Andreas, 34212 Melsungen (DE); SCHUMACHER, Volker, 88339 Bad Waldsee (DE); Dr. KLUG, Robert, 79356 Eichstetten (DE); SIPPEL, Martin, 34212 Melsungen (DE)
(74) Vertreter: Klingseisen, Franz
(86) Internationale Anmeldenummer: PCT/EP2009/002615
(87) Internationale Veröffentlichungsnummer: WO 2009/124751

(56) Entgegenhaltungen:
- EP-A- 0 522 737
- DE-U1- 20 001 205
- US-A- 5 030 207
- US-A1- 2002 055 715
- US-A1- 2006 116 660

## Beschreibung

Die Erfindung betrifft eine Spinalkanüle mit einem Kanülenansatz, der einen transparenten Bereich zur Beobachtung der durch den Kanülenansatz fließenden Rückenmarks-Flüssigkeit (Liquor bzw. cerebrale spinale Flüssigkeit CSF) aufweist.

Spinalkanülen werden zur Punktion des Spinalraums verwendet, wobei der Liquor-Rückfluss kontrolliert werden muss, damit die korrekte Positionierung der Kanülenspitze im Subarachnoidalraum kontrolliert werden kann. Die Schwierigkeit liegt dabei darin, dass der wasserklare und farblose Liquor optisch nicht leicht im Kanülenansatz erkennbar ist.

Aus US-6 656 161 ist eine Spinalkanüle mit einem transparenten Bereich am Kanülenansatz bekannt, an dem eine Vergrößerungslinse ausgebildet ist, damit man den Rückfluss des Liquors erkennen kann.

Aus EP-682 954 ist eine Spinalkanüle mit transparentem Griffteil bekannt, in dem eine sich zum Kanülenende hin verjüngende Liquor-Kontrollkammer ausgebildet ist, die eine im Wesentlichen rechteckige Querschnittsform mit paarweise keilförmig zueinander verlaufenden ebenen Wandflächen aufweist. Bei dieser Ausgestaltung soll sichergestellt werden, dass der Liquor-Rückfluss innerhalb der Liquor-Kontrollkammer ohne störende Reflexe und Lichtbrechungen gut erkennbar ist.

Der Erfindung liegt die Aufgabe zugrunde, eine Spinalkanüle der eingangs angegebenen Art so auszubilden, dass der Liquor-Rückfluss optisch besser kontrolliert werden kann.

Es wird am, auf oder im transparenten Bereich des Kanülenansatzes eine Lichtstrahlen brechende bzw. reflektierende Einrichtung, wie beispielsweise ein Dach- oder Tripelprisma, vorgesehen. Solange kein Liquor-Rückfluss stattfindet und sich nur Luft im Kanülenansatz befindet, wird durch die Lichtstrahlen reflektierende bzw. brechende Einrichtung ein anderes optisches Bild für den Anwender wiedergegeben als bei Liquor-Rückfluss, wenn sich Liquor im Kanülenansatz befindet, wobei der Liquor die Brechung bzw. Reflexion der Lichtstrahlen verändert und sich dadurch ein anderes optisches Bild ergibt. Auf diese Weise kann sehr deutlich festgestellt werden, ob ein Liquor-Rückfluss stattfindet oder nicht.

Wenn die Lichtstrahlen reflektierende Einrichtung als Prisma ausgebildet wird, ergeben sich einfache Gestaltungsmöglichkeiten der Spinalkanüle, wobei das Prisma auf dem Außenumfang des Kanülenansatzes bzw. des transparenten Bereichs oder auf dem Innenumfang einer Beobachtungskammer im Kanülenansatz ausgebildet werden kann.

Zur Verbesserung der Erkennung kann dem Außenprisma in Betrachtungsrichtung wenigstens eine gekrümmte Fläche des Hohlraumes oder des Außenumfangs des Kanülenansatzes als Lichtstrahlen brechende Einrichtung zugeordnet sein.

Nach einer weiteren Ausführungsform können in Achsrichtung versetzt zueinander Reihen von Außenprismen längs des Umfangs ausgebildet werden, wobei die Prismen in jeder Reihe in Umfangsrichtung einen Abstand voneinander haben. Auf diese Weise ergibt sich im Bereich mehrerer Prismen eine Änderung der Brechung bzw. Reflexion der Lichtstrahlen, wenn sich Liquor im Beobachtungsbereich befindet.

Dieser Effekt wird auch erreicht, wenn mehrere Innenprismen nebeneinander auf dem Innenumfang der Beobachtungskammer im Kanülenansatz ausgebildet sind.

Nach einer anderen Ausgestaltung kann wenigstens eine Prismenfläche den Hohlraum bzw. die Beobachtungskammer im Kanülenansatz begrenzen, um eine Veränderung des optischen Bildes zu erreichen, wenn Liquor in die Beobachtungskammer gelangt.

Vorteilhafterweise kann das Prisma oder können die Prismen an der Spinalkanüle angeformt sein, um die Herstellung kostengünstig zu gestalten.

Zur Verbesserung der Lichtbrechung bzw. Lichtreflexion kann die Lichtstrahlen reflektierende Einrichtung als spiegelnde Fläche ausgebildet werden, die auf dem Außenumfang im transparenten Bereich des Kanülenansatzes aufgebracht wird, wobei die spiegelnde Seite dem Hohlraum zugewandt ist.

Die Handhabung der Spinalkanüle wird dadurch erleichtert, dass die Spinalkanüle von einer Griffmuffe mit wenigstens einer Aussparung auf dem Umfang umgeben ist, die über der Lichtstrahlen brechenden bzw. reflektierenden Einrichtung angeordnet ist.

Die erfindungsgemäße Ausgestaltung kann auch an einem anderen Hohlkörper, insbesondere an einem Schlauch vorgesehen werden, der wenigstens einen transparenten Wandabschnitt aufweist, wobei im Bereich des transparenten Wandabschnitts eine Lichtstrahlen brechende bzw. reflektierende Einrichtung zur optischen Erkennung von Flüssigkeit in dem Hohlraum bzw. Schlauch vorgesehen ist. Hierbei kann die Lichtstrahlen brechende bzw. reflektierende Einrichtung in einer Form ausgebildet sein, wie sie im Zusammenhang mit Spinalkanülen beschrieben sind.

Die Erfindung umfasst auch Kombinationen von einzelnen oder mehreren der zuvor beschriebenen Merkmale. Z. B. können ein Innen- und ein Außenprisma im Beobachtungsbereich vorgesehen werden wie auch ein Prisma oder mehrere Prismen in Verbindung mit einer spiegelnden Fläche verwendet werden können. Durch Kombinationen der beschriebenen Ausführungsformen kann die Liquor-Erkennung verbessert werden.

Die Erfindung wird beispielsweise anhand der Zeichnung näher erläutert. Es zeigen:
- Fig. 1: schematisch eine Spinalkanüle mit beispielsweise rundem Querschnitt des Kanülenansatzes,
- Fig. 2: schematisch einen Querschnitt einer Spinalkanüle mit einem Prisma im Innenbereich,
- Fig. 3: schematisch einen Querschnitt einer Spinalkanüle mit einem Prisma im Außenbereich,
- Fig. 4: ein Dachprisma mit einfallenden, reflektierten und ausfallenden Lichtstrahlen,
- Fig. 5: ein Tripelprisma mit einfallenden, reflektierten und ausfallenden Lichtstrahlen,
- Fig. 6: schematisch einen Längsschnitt einer Spinalkanüle mit mehreren auf einem Kreis, nebeneinander angeordneten Dachprismen im Innenbereich,
- Fig. 7: schematisch einen Längsschnitt einer Spinalkanüle mit zwei versetzt gegenüberliegenden Tripelprismen im Außenbereich,
- Fig. 8: schematisch einen Längsschnitt einer Spinalkanüle mit mehreren Dachprismen im Innenbereich und einem Griffbereich mit durchbrochenen Sichtfenster,
- Fig. 9: schematisch einen Querschnitt einer Spinalkanüle mit einer spiegelnden Schicht im Außenbereich anstelle eines Außenprisma, und
- Fig. 10: schematisch einen Längsschnitt einer Spinalkanüle mit zwei versetzt gegenüberliegenden spiegelnden Schichten im Außenbereich anstelle von Außenprismen.

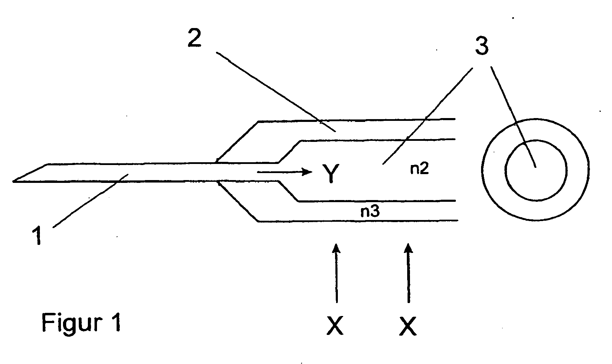

Fig. 1 zeigt in einem schematischen Längsschnitt eine Spinalkanüle mit einer in einem Kanülenansatz 2 befestigten Kanüle 1. Der Kanülenansatz 2 weist wenigstens einen transparenten Bereich bzw. ein Sichtfenster mit Brechungsindex n3 auf, durch den bzw. durch das der Hohlraum 3 mit Brechungsindex n2 (Luft) im Kanülenansatz beobachtet werden kann. Dieser Hohlraum 3 bildet eine Beobachtungskammer. Die optische bzw. visuelle Beobachtung erfolgt in den Figuren in Richtung der Pfeile X. Der Eintritt des Liquor in den Hohlraum 3 erfolgt in den Figuren über die Kanüle 1 in Richtung des Pfeils Y.

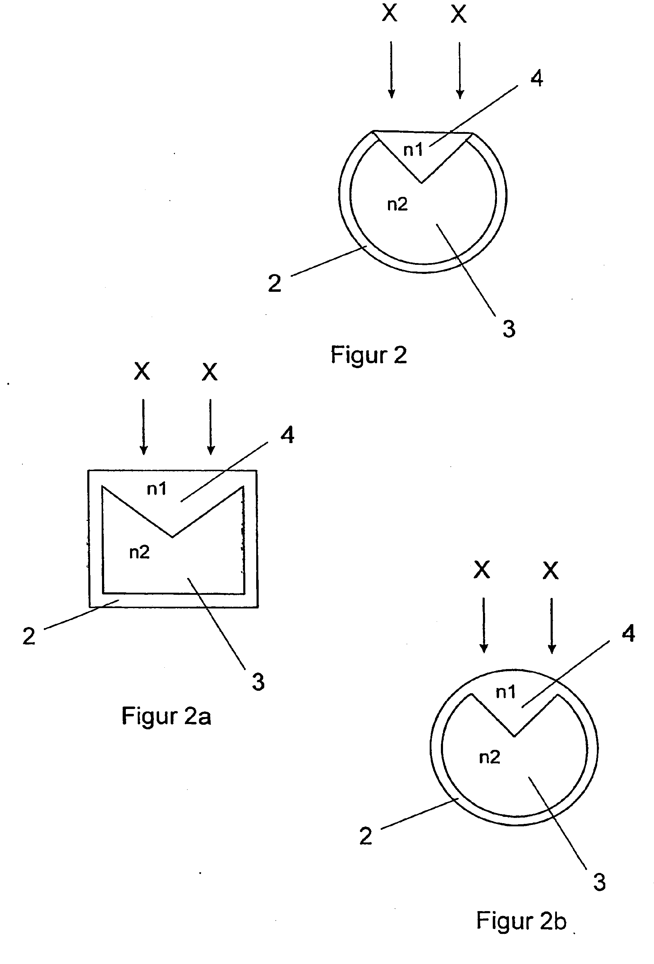

Fig. 2 zeigt ein Ausfiihrungsbeispiel, bei dem im Bereich des Sichtfensters ein Prisma 4 mit Brechungsindex n1 so angebracht ist, dass sich das Prsima 4, im Weiteren Innenprisma genannt, mit den beiden giebelförmigen Flächen nach innen in den Hohlraum 3 erstreckt. Bei dieser Ausfiihrungsform hat der Kanülenansatz 2 eine runde bzw. hohlzylindrische Querschnittsform, wobei das Innenprisma 4 so in den Querschnitt des Kanülenansatzes integriert ist, dass die Basisfläche des Prismas auf dem Außenumfang des Kanülenansatzes 2 eine Abflachung bildet. Solange sich Luft in der Beobachtungskammer 3 des Kanülenansatzes 2 befindet, wird bei Beobachtung durch das Prisma 4 längs der Pfeile X ein deutlich erkennbarer metallischer Glanz im Bereich des Innenprismas wahrgenommen. Sobald die Luft in der Beobachtungskammer 3 durch den Liquor verdrängt wird, verschwindet dieser metallische Glanz und der Bereich erscheint transparent.

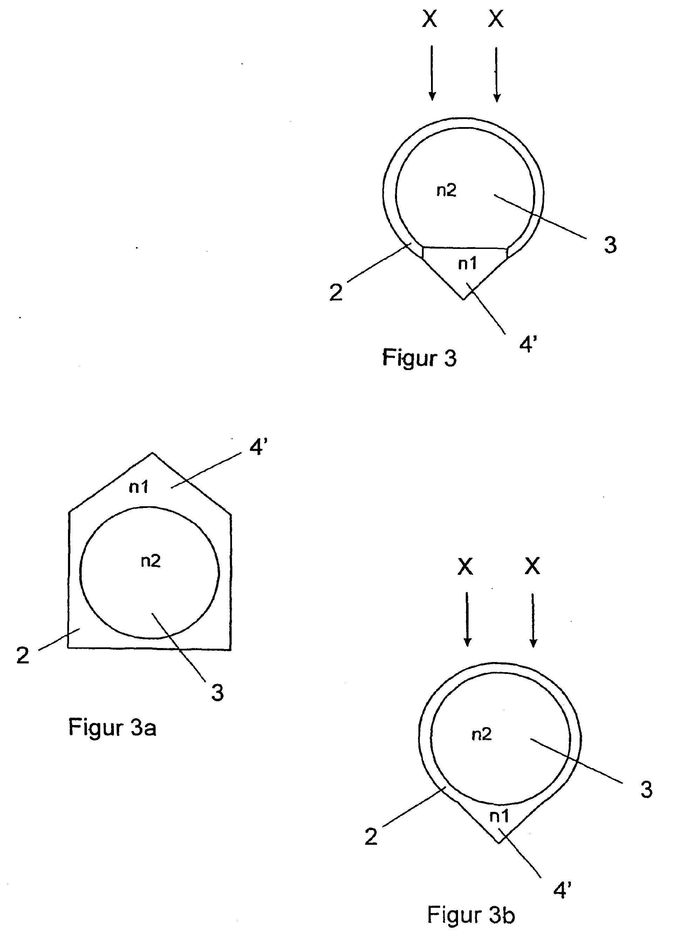

Bei der in Fig. 3 schematisch wiedergegebenen Ausführungsform ist das Prisma 4' mit Brechungsindex n1 so angeordnet, dass die beiden giebelförmigen Flächen nach außen weisen und die Basisfläche des Prismas, im Weiteren Außenprisma genannt, auf dem Innenumfang des Hohlraums 3 eine Abflachung bildet. Hierdurch ergibt sich ein anderes Bild bei der Beobachtung durch ein Sichtfenster längs der Pfeile X, das diametral gegenüberliegend zum Prisma 4' ausgebildet ist. Solange Luft in der Beobachtungskammer 3 vorhanden ist, ist nur ein sehr schwer wahrnehmbarer kleiner metallisch glänzender Fleck im Bereich des Prismas 4' erkennbar. Dieser glänzende Reflex wird größer und tritt erst dann deutlich auf, wenn Liquor die Luft in der Beobachtungskammer 3 verdrängt.

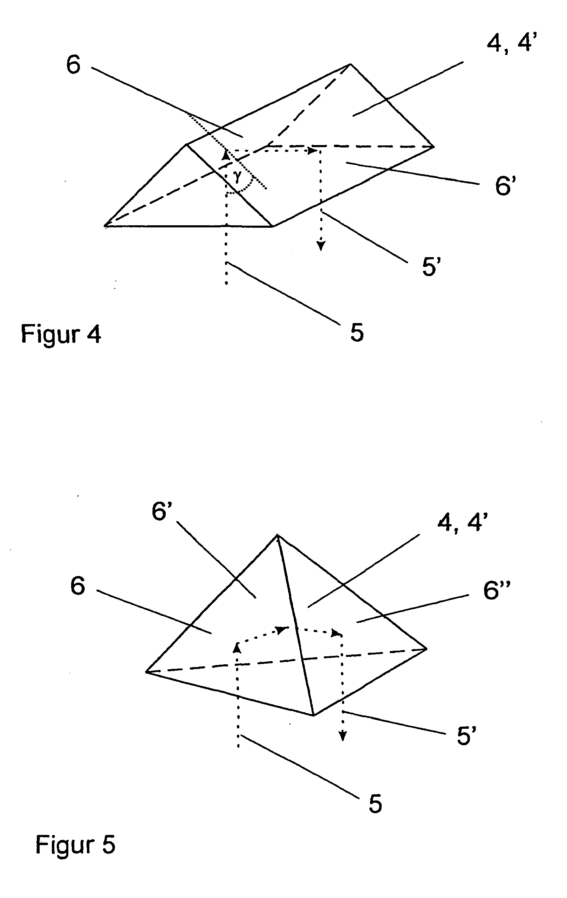

Fig. 4 zeigt ein rechtwinklig-gleichschenkliges Prisma, im Weiteren als Dachprisma bezeichnet, das als Innenprima 4 oder Außenprisma 4' eingesetzt werden kann. Der Brechungsindex n1 dieses Prismas kann beispielsweise zwischen 1.42 bis 1.62 liegen und der Brechungsindex n2 der Prismenumgebung bei ca. 1, wenn das Prisma von Luft umgeben ist. Ein einfallender Lichtstrahl 5 erfahrt unter diesen Bedingungen an den Prismenflächen 6 und 6' Totalreflexion, wenn dieser unter einem Winkel γ von 45° auf die Prismenflächen 6 und 6' fällt. Der ausfallende Lichtstrahl 5' ist damit um 180° zum einfallenden Lichtstrahl 5 gedreht bzw. gespiegelt worden. Ein Beobachter der in Richtung des einfallenden Lichtstrahls blickt, wird unter diesen Umständen einen Lichtreflex wahrnehmen. Die Bedingungen für Totalreflexion bei γ = 45° werden aufgehoben, wenn sich der Brechungsindex n2 von ca. 1 auf größer als 1.32 ändert. Dies ist bei Liquor an den Prismenflächen der Fall. Ein Beobachter der unter diesen Umständen in Richtung des einfallenden Lichtstrahls 5 blickt, kann keinen Lichtreflex wahrnehmen.

Fig. 5 zeigt ein Tripelprisma, das als Innenprisma 4 oder Außenprisma 4' eingesetzt werden kann. Auch hier gelten die gleichen Bedingungen wie für das Dachprisma in Fig. 4 mit dem Unterschied, das es an drei Stellen zur Totalreflexion bzw. nicht zur Totalreflexion kommt, je nach Brechungsindex n2 der Tripelprismaumgebung an den drei Flächen 6, 6' und 6".

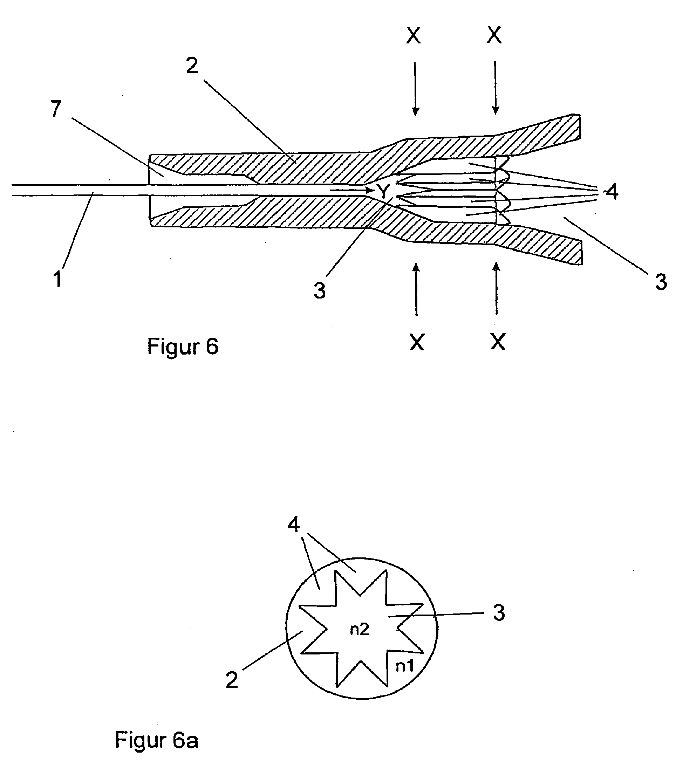

Fig. 6 zeigt in einem schematischen Längsschnitt eine Spinalkanüle mit transparentem Kanülenansatz 2 aus Kunststoff mit Brechungsindex n3, bei dem die Kanüle 1 durch einen mit Kleber gefüllten Spalt 7 am Kanülenansatz befestigt ist und bei dem mehrere Innenprismen 4, gebildet aus Dachprismen mit spitzabgeflachten Stirnflächen, zirkularsymmetrisch auf dem Innenumfang im Bereich des Sichtfensters angeordnet sind. Die Dachprismen 4 bestehen aus dem gleichen Kunststoffmaterial mit Brechungsindex n1 wie der Kanülenansatz 2 (n1 = n3). Diese Anordnung ermöglicht eine Erkennung des Liquor-Rückfluss von allen Seiten bei Blickrichtung senkrecht zur Längsachse der Spinalkanüle.

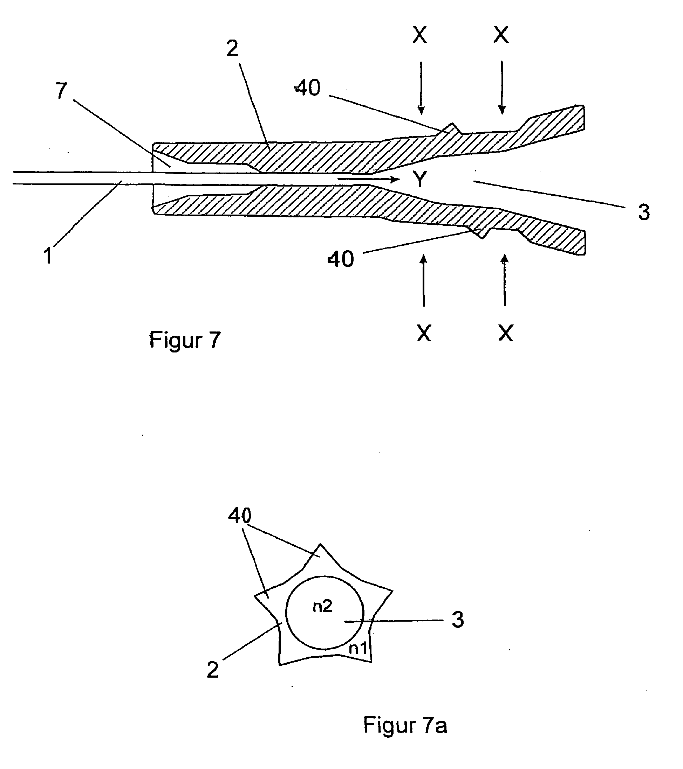

Fig. 7 zeigt in einem schematischen Längsschnitt eine Spinalkanüle mit transparentem Kanülenansatz 2 aus Kunststoff mit Brechungsindex n3, bei dem die Kanüle 1 durch einen mit Kleber gefüllten Spalt 7 am Kanülenansatz befestigt ist und bei dem das Außenprisma aus zwei zueinander versetzten Reihen 40 von senkrecht zur Zeichnungsebene hintereinander angeordneten Tripelprismen (Fig. 5) auf dem Außenumfang des Kanülenansatz aufgebaut ist. Die Tripelprismen bestehen aus dem gleichen Kunststoffmaterial mit Brechungsindex n1 wie der Kanülenansatz 2 (n1 = n3). Anstelle der Tripelprismen können auch Dachprismen (Fig. 4) bei dieser Anordnung verwenden. Die einzelnen Prismen einer Reihe haben vorzugsweise einen derartigen Abstand voneinander, dass bei Beobachtung längs der Pfeile X durch diesen Abstand bzw. durch die Lücke hindurch das auf der gegenüber liegenden Seite angeordnete Prisma der selben oder auch der anderen Reihe betrachtet werden kann.

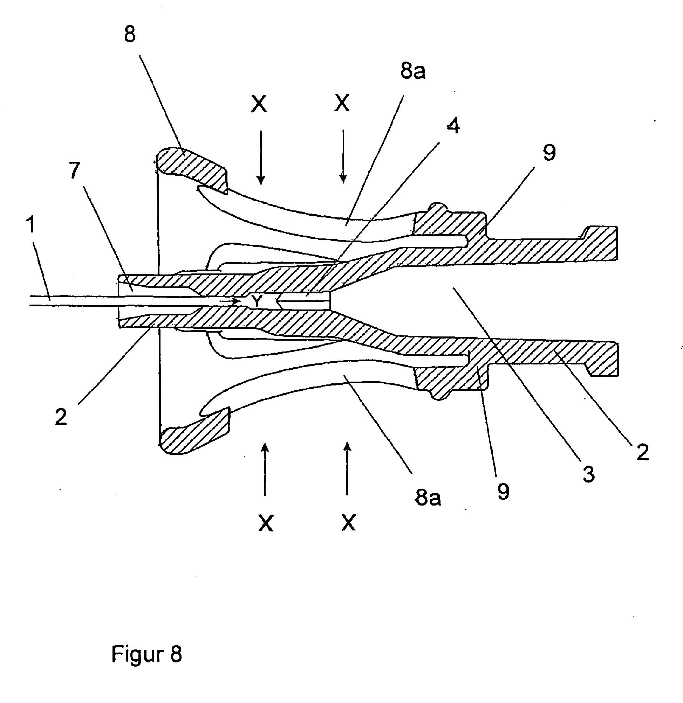

Fig. 8 zeigt einen schematischen Längsschnitt einer Spinalkanüle ähnlich Fig. 6, wobei der Kanülenansatz 2 zusätzlich mit einer Haltevorrichtung 8 zur Verbesserung der Haptik der Spinalkanüle versehen ist. Diese Haltevorrichtung ist als konische Griffmuffe 8 um den Kanülenansatz 2 ausgebildet, die am engeren Ende bei 9 mit dem Kanülenansatz verbunden ist. Die Griffmuffe 8 weist Aussparungen 8a im Bereich des Sichtfensters des Kanülenansatz 2 auf, so dass in Richtung der Pfeile X der Liquor-Rückfluss mittels des Innenprismas 4 festgestellt werden kann, das der Aussparung 8a gegenüberliegt. Die Aussparungen 8a lenken den Blick des Beobachters auf das darunter liegende Sichtfenster mit Prisma. Wenn beispielsweise auf dem Umfang der Griffmuffe 8 vier Aussparungen 8a vorgesehen werden, werden auf dem Innenumfang des Kanülenansatzes vier Prismen 4 den Aussparungen 8a gegenüberliegend ausgebildet. Eine solche Griffmuffe 8 kann an der Spinalkanüle angeformt oder als getrenntes Element aufgesetzt und z. B. durch Kleben befestigt sein.

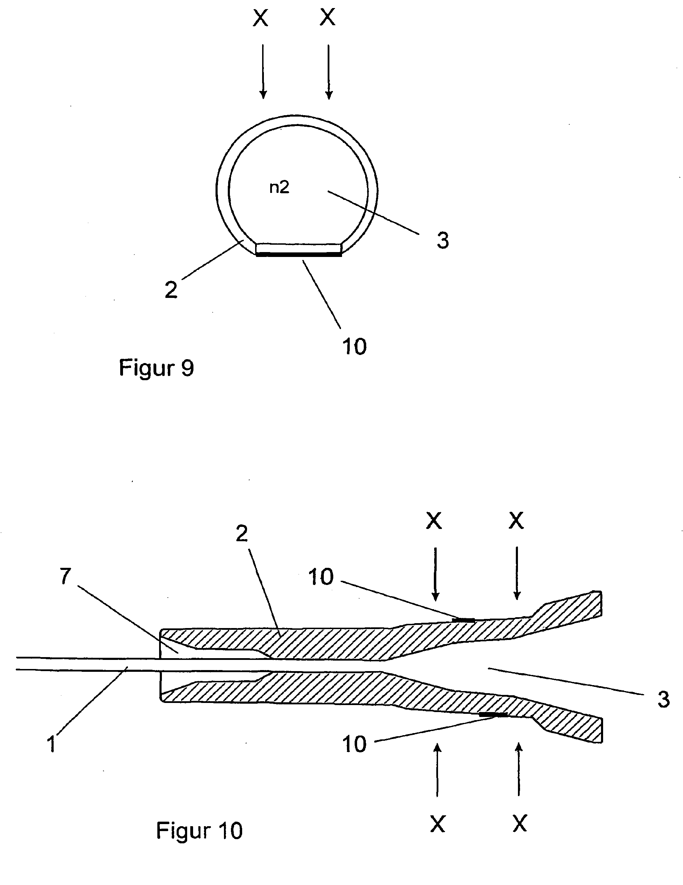

Fig. 9 zeigt eine andere Ausfiihrungsform nach der Erfindung, bei der anstelle eines Außenprismas eine spiegelnde Schicht 10 z. B. aus Silber oder Aluminium aufgebracht ist. Anstelle einer solchen spiegelnden Schicht 10 kann auch ein optisches Reflexionsgitter vorgesehen sein.

Fig. 10 zeigt einen Längsschnitt durch einen Kanülenansatz, wobei entsprechend Fig. 7 auf dem Außenumfang in Achsrichtung versetzt zwei Reihen von spiegelnden Flächen 10 mit entsprechendem Abstand zwischen den Flächen aufgebracht sind.

Durch die Änderung des metallischen Glanzes in Beobachtungsrichtung X im Sichtfenster wird bei allen Ausführungsformen nach den Fig. 2 und 3 sowie 6, 7 und 8 das Vorhandensein des ansonsten sehr schwer erkennbaren transparenten Liquors im Hohlraum 3 des Kanülenansatz 2 deutlich feststellbar. Bewirkt wird dieser optische Effekt durch die Änderung des Brechungsindexes von ca. 1 bei Luft im Hohlraum 3 auf 1.32 bis 1.35 bei Liquor im Hohlraum 3.

Bei einem Innenprisma 4 in Form eines Dach- oder Tripelprismas wird beim Vorhandensein von Luft im Hohlraum 3 ein deutlicher metallischer Glanz im Bereich des Innenprismas 4 wahrgenommen. Dies wird bewirkt durch den Effekt der Totalreflexion wie in den Figuren 4 und 5 mit Luft als Prismenumgebung dargestellt. Der Einfallswinkel γ, ab dem Totalreflexion stattfindet, wird bestimmt durch γ = arcsin (n2 / n1), wobei n2 der Brechungsindex der Prismenumgebung im Hohlraum 3 (Wasser oder Liquor) ist und n1 dem Brechungsindex des Prismas 4, 4'. Alle Lichtstrahlen, die mit einem größeren Winkel γ auf die Seitenflächen des Prismas in Fig. 4 oder 5 fallen, unterliegen dem Effekt der Totalreflexion und werden an der Seitenfläche 6 des Prismas reflektiert. Da bei einem Dachprisma eine Totalreflexion zweimal und beim Tripelprisma dreimal auftritt, wird vorzugsweise ein Auftreffwinkel γ von ca. 45° gewählt, denn nur so erfolgt eine Umkehrung bzw. Spiegelung des Lichtstrahls durch das Prisma 4, 4' mittels Totalreflexion..

Für ein Prisma aus einem gängigen transparenten Kunststoff liegt der Brechungsindex zwischen 1.42 bis 1.6. Der Brechungsindex von Luft liegt bei ca. 1 und der des Liquors zwischen 1,32 bis 1,35. Bei Luft in Hohlraum 3 ergibt sich ein Winkel γ zwischen 44,7° bis 38,1 °, vorzugsweise sollte der Winkel γ klein gewählt werden, um einen möglichst großen Bereich für die Totalreflexion zuzulassen. Transparente Kunststoffe, die diese Bedingungen mit einen Brechungsindex von ca. 1,59 erfüllen, sind Polycarbonate (PC), aber auch andere transparente Polymere wie PMMA, CMMA, COC, SAN, PS und ABS sind geeignet. Für ein Prisma aus PC gilt für Luft γ > 38,9° und für Liquor γ > 56,1° bis 58,1 °. Da eine Lichtstrahlumkehrung bzw. Reflexion bei einen Dachprisma und Tripelprisma nur unter einem Winkel von 45° erfolgt, ist bei Luft im Hohlraum 3 diese Bedingung erfüllt und bei Liquor im Hohlraum 3 nicht. Für einen Beobachter, der vorzugsweise nahezu parallel zum einfallenden Lichtstrahl in Pfeilrichtung X gemäß den Figuren 2, 6 und 8 auf das Prisma 4 blickt, zeigt sich bei Luft im Hohlraum ein metallischer Glanz durch die Spiegelung des Lichtstahls und bei Liquor im Hohlraum 3 entfällt diese Spiegelung und der Bereich erscheint transparent.

Bei einem Außenprisma 4' in Form eines Tripelprismas wird beim Vorhandensein von Luft im Hohlraum 3 ein kaum wahrnehmbarer kleiner metallisch glänzender Fleck im Bereich des Außenprismas wahrgenommen. Dies wird durch Totalreflexion an den Seitenflächen 6, 6' und 6" des Tripelprismas bewirkt, wie in Fig. 3 dargestellt, und durch einen Beobachter wahrgenommen, der in Richtung der Pfeile X durch den transparenten Teil des Kanülenansatz bzw. des Sichtfensters und des Hohlraums hindurch auf das Außenprisma 4 blickt. Der Fleck ist sehr klein und kaum wahrnehmbar, da die Lichtstrahlen längst der Pfeile X an den gekrümmten Oberflächen des Kanülenansatz 2 gebrochen werden. Dies geschieht beim Übergang von Luft in den Kanülenansatz 2 und dann weiter beim Übergang vom Kanülenansatz 2 in den Hohlraum 3. Das auf das Außenprisma fallende Licht und das vom Außenprisma gespiegelte Licht wird so jeweils an den Grenzflächen Luft/Kanülenansatz und Kanülenansatz / Luft gebrochen. Durch diese mehrmalige starke Brechung erscheint nur ein kleiner Fleck mit metallischem Glanz. Der Effekt der Brechung wird beschrieben durch das Gesetz von Snellus und für die Brechung an der Grenzfläche Kanülenansatz / Luft gilt sin(β) = sin(α) n1 / n2 wobei n1 der Brechungsindex der Kanülenansatz und n2 der des Mediums im Hohlraums 3 ist. Der Winkel α ist der Einfallswinkel auf die Grenzfläche Kanülenansatz 2 / Hohlraum 3 und der Winkel β der Ausfallswinkel. Befindet sich Luft im Hohlraum 3 mit n2 ca. 1, ist der Ausfallswinkel β größer als wenn sich Liquor mit einem Brechungsindex von 1.32 bis 1.36 im Hohlraum 3 befindet. Bei Luft im Hohlraum 3 erfolgt eine stärkere Brechung als bei Liquor im Hohlraum 3 an der Grenzfläche Kanülenansatz / Hohlraum 3. Wird die Brechung der Lichtstrahlen verringert durch das Vorhandensein von Liquor im Hohlraum 3, so erscheint der bei Luft in Hohlraum 3 nur kaum wahrnehmbare Fleck mit metallischen Glanz nun viel größer und deutlicher. Vorteilhaft kann dieser Effekt ausgelegt werden, wenn die Krümmung an der Grenzfläche Luft / Kanülenansatz 2 sehr gering ist, die an der Grenzfläche Kanülenansatz 2 / Hohlraum 3 sehr groß und der Brechungsindex des Kanülenansatz n 1 sehr nahe an dem des Liquors liegt. Vorteilhaft ist ein Außenprisma wie in Figur 7 dargestellt aus dem gleichen Kunststoffmaterial wie der Kanülenansatz. Eine solche Anordnung kann in einem Herstellungsprozess wie z.B. Kunststoffspritzguss hergestellt werden ohne weitere Verarbeitungsschritte.

Anstelle eines Außenprimas 4" in Form eines Tripel- oder Dachprimas kann auch eine spiegelnde Schicht 10 aufgebracht werden. Solch eine Schicht kann wie bei üblichen Spiegeln aus einer dünnen Schicht aus Aluminium, Silber, einer Abfolge von hoch und niedrig brechenden Schichten oder ähnlichen bestehen. Diese dünne Schicht sollte in ihrer Fläche dem der Basisfläche der beschriebenen Tripel- und Dachprismen entsprechen. Die Funktion der spiegelnden Sicht ist die selbe wie bei einem Außenprisma. Der einfallende Lichtstrahl wird mittels der beiden Vorrichtungen reflektiert. Dabei ist es für die Anwendung zur Liquor-Erkennung nicht ausschlaggebend, dass bei einem Prisma der reflektierte Lichtstrahl zum einfallenden Lichtstrahl einen Versatz erfährt und bei einer spiegelnden Sicht nicht. Auch bei einer spiegelnden Sicht gelten die gleichen optischen Effekte für Lichtstrahlbrechung und -reflexion wenn sich Luft oder Liquor im Hohlraum 3 befindet, wie bei einem Außenprisma. Anstelle der reflektierenden Schicht oder des Außenprisma kann auch eine Beugungsgitter mit Reflexions Eigenschaften verwendet werden.

Die Figuren 6 und 8 zeigen weitere vorteilhafte Ausführungsformen mit Innenprisma. Dabei wird durch die reihenförmige Anordnung mehrerer Prismen auf der innenfläche des Kanülenansatz 2 eine Beobachtung des Liquor-Rückfluss von allen Seiten senkrecht auf die Spinalkanüle erreicht. Figur 8 zeigt eine besonders vorteilhafte Ausführungsforrn, bei der der Anwender durch die als Fenster wirkenden Aussparungen 8a in der Griffmuffe 8 in seiner Blickrichtung geführt wird und die Krümmung an der Grenzfläche Luft / Kanülenansatz 2 im Bereich des Sichtfensters sehr gering gehalten ist, bspw. durch eine Polygonfläche in der Querschnittsansicht.

Figur 7 zeigt eine weitere vorteilhafte Ausführungsform mit Außenprisma. Tripel- oder Dachprismen sind in zwei nebeneinander angeordneten Reihen auf dem Umfang des transparenten Kanülenansatz 2 angeordnet. Die einzelnen Prismen einer Reihe haben vorzugsweise einen derartigen Abstand voneinander, dass bei Beobachtung längs der Pfeile X durch diesen Abstand bzw. durch die Lücke hindurch das auf der gegenüber liegenden Seite angeordnete Prisma der selben oder auch der anderen Reihe betrachtet werden kann. Diese Anordnung ermöglicht ähnlich wie bei Fig. 6 und 8 eine Erkennung des Liquor-Rückfluss von allen Seiten senkrecht zur Längsachse der Spinalkanüle

In den Fig. 2 und 3 ist das Prisma jeweils so in der Querschnittsansicht des Kanülenansatzes eingesetzt, dass die Basisfläche des Prismas eine Abflachung auf dem Innen- oder Außenumfang des Kanülenansatzes bildet. Es ist aber auch möglich, das Prisma so am Kanülenansatz anzuformen, dass beim Innenprisma 4 der Außenumfang des Kanülenansatzes 2 einen Vollkreis bildet, so dass die Basisfläche des Prismas, die in Fig. 2 eine Abflachung auf dem Außenumfang bildet, eine gekrümmte Form hat. In gleicher Weise kann bei dem Außenprisma nach Fig. 3 der Innenumfang des Hohlraums 3 kreisförmig sein, sodass nur die Seitenflächen des Prismas nach außen aus dem ansonsten runden Querschnitt des Kanülenansatzes 2 vorstehen.

Anstelle des wiedergegebenen runden Querschnitts der Beobachtungskammer 3 im Kanülenansatz 2 kann auch eine andere Querschnittsform vorgesehen werden, insbesondere wenn die Art der lichtbrechenden Einrichtung anders ausgebildet wird.

Der Kanülenansatz 2 kann im Bereich der Beobachtungskammer 3 einen kreisförmigen, eliptischen, polygonalen oder rechteckigen Querschnitt haben.

Bei Verwendung eines Außenprismas 4' liegt diesem in Betrachtungsrichtung X (Fig. 3) eine gekrümmte Fläche des Hohlraums 3 und/oder des Außenumfangs des Kanülenansatzes 2 gegenüber, wobei die gekrümmte Fläche die Lichtstrahlen brechende Einrichtung bildet. Wird ein Innenprisma 4 (Fig. 2) verwendet, so kann die den giebelförmigen Flächen des Innenprismas gegenüberliegende Begrenzungsfläche des Hohlraums 3 in verschiedener Weise gestaltet sein, beispielsweise kann der Hohlraum 3 auch einen rechteckigen oder quadratischen Querschnitt haben, wie dies Fig. 2a zeigt.

Fig. 2a zeigt als Ausfiihrungsbeispiel ein Innenprisma 4 bei einem möglichen Querschnitt des Hohlraums bzw. Beobachtungsraums 3, der auf dem Außenumfang quadratisch und auf dem Innenumfang polygon ausgebildet ist.
Fig. 2b zeigt als Ausführungsbeispiel einen Querschnitt einer schlauchförmigen Beobachtungskammer 3 mit Innenprisma 4.

Fig. 3a zeigt eine mögliche Ausführungsform mit einem Außenprisma 4', wobei der Außenumfang polygon oder rechteckig gestaltet und der Innenumfang des Hohlraums bzw. der Beobachtungskammer 3 rund ausgebildet ist, sodass in Betrachtungsrichtung X des Außenprismas 4' diesem wenigstens eine gekrümmte Fläche zugeordnet ist.
Fig. 3b zeigt einen Schlauchquerschnitt mit Außenprisma 4' als weiteres Ausführungsbeispiel.

Bei den Ausführungsformen nach den Fig. 2 und 3 erstreckt sich das Prisma in Achsrichtung des Kanülenansatzes 2. Es ist aber auch möglich, schräg oder quer zur Achse des Kanülenansatzes ein Prisma oder mehrere Prismen nebeneinander anzuordnen.

Bei den beschriebenen Ausfiihrungsformen ist die lichtbrechende bzw. reflektierende Einrichtung vorzugsweise am Kanülenansatz 2 angeformt. Es ist aber auch möglich, insbesondere bei der Ausführungsform nach Fig. 3, das Prisma 4' als gesondertes Bauteil am Kanülenansatz 2 beispielsweise durch Kleben anzubringen.

Vorzugsweise besteht der gesamte Kanülenansatz 2 aus transparentem Kunststoffmaterial.

Die spiegelnde Schicht 10 kann auch auf dem konvex gekrümmten Außenumfang des Kanülenansatzes 2 aufgebracht sein, wobei die spiegelnde Seite dem Hohlraum bzw. der Beobachtungskammer zugewandt ist.

Mit der beschriebenen Anordnung von Innen- und Außenprisma kann nicht nur der Liquor-Rückfluss in einem Spinalkanülenansatz festgestellt werden, sondern generell das Vorhandensein oder nicht Vorhandensein von transparenten Flüssigkeiten in einem Hohlraum 3 z.B. in anders gearteten Aspirationskanülen oder auch in einem Schlauch. Mögliche Anwendungen bei einem Schlauch sind z.B. auch die Flüssigkeitserkennung in Infusionsüberleitunssystemen bzw. die Luftblasenerkennung in diesen.

Fig. 6a zeigt einen Querschnitt durch die Beobachtungskammer in Fig. 6 mit auf dem Innenumfang angeordneten Innenprismen 4.

Fig. 7a zeigt einen Querschnitt durch den Kanülenansatz der Fig. 7 längs einer Reihe der Außenprismen 40. Die Außenprismen 40 der benachbarten Prismenreihe sind in Umfangsrichtung vorzugsweise derart versetzt angeordnet, dass in der Ansicht der Fig. 7a die Außenprismen 40 zwischen denen der in Fig. 7a wiedergegebenen Prismen angeordnet sind.

## Patentansprüche

1. Spinalkanüle mit einem Kanülenansatz (2), der einen transparenten Bereich zur Beobachtung des durch einen Hohlraum (3) des Kanülenansatzes fließenden Liquors aufweist, wobei in oder an dem transparenten Bereich des Kanülenansatzes (2) eine Lichtstrahlen brechende bzw. reflektierende Einrichtung vorgesehen ist. **dadurch gekennzeichnet daß** die Lichtstrahlen reflektierende Einrichtung als Prisma (4, 4') ausgebildet ist.

2. Spinalkanüle nach Anspruch 1, wobei das Prisma (4') auf dem Außenumfang des Kanülenansatzes (2) bzw. des transparenten Bereichs angebracht ist.

3. Spinalkanüle nach Anspruch 2, wobei in Achsrichtung versetzt zueinander Reihen von Außenprismen (40) längs des Umfangs ausgebildet sind und die Prismen in jeder Reihe in Umfangsrichtung einen Abstand voneinander haben.

4. Spinalkanüle nach Anspruch 2 oder 3, wobei dem Außenprisma (4') in Betrachtungsrichtung (X) wenigstens eine gekrümmte Fläche des Hohlraumes (3) oder des Außenumfangs des Kanülenansatzes (2) als Lichtstrahlen brechende Einrichtung zugeordnet ist.

5. Spinalkanüle nach Anspruch 1, wobei das Prisma (4) auf dem Innenumfang einer Beobachtungskammer (3) im Kanülenansatz (2) ausgebildet ist.

6. Spinalkanüle nach Anspruch 5, wobei mehrere Innenprismen (4) nebeneinander auf dem Innenumfang der Beobachtungskammer (3) im Kanülenansatz (2) ausgebildet sind.

7. Spinalkanüle nach einem der Ansprüche 1 bis 6, wobei wenigstens eine Prismenfläche den Hohlraum bzw. die Beobachtungskammer (3) im Kanüknansatz(2) begrenzt.

8. Spinalkanüle nach einem der Ansprüche 1 bis 7, wobei das Prisma (4,4') oder die Prismen an der Spinalkanüle angeformt ist bzw. sind. mit einem Kanülenansatz (2), der einen transparenten Bereich zur Beobachtung des durch einen Hohlraum (3) des Kanülenansatzes fließenden Liquors aufweist, wobei in oder an dem transparenten Bereich des Kanülenansatzes (2) eine Lichtstrahlen brechende bzw. reflektierende Einrichtung vorgesehen ist, **dadurch gekennzeichnet daß**

9. Spinalkanüle die Lichtstrahlen reflektierende Einrichtung als spiegelnde Fläche (10) ausgebildet ist, die auf dem Außenumfang im transparenten Bereich des Känülenansatzes aufgebracht ist, wobei die spiegelnde Seite dem Hohlraum (3) zugewandt ist.

10. Spinalkanüle nach einem der vorhergehenden Ansprüche, wobei die Spinalkanüle von einer Griffmuffe (8) mit wenigstens einer Aussparung (8a) auf dem Umfang umgeben ist, die über der Lichtstrahlen brechenden bzw. reflektierenden Einrichtung angeordnet ist.

11. Hohlkörper, insbesondere Schlauch, mit wenigstens einem transparenten Wandabschnitt, wobei im Bereich des transparenten Wandabschnitts eine Lichtstrahlen brechende bzw. reflektierende Einrichtung(4, 4', 10) zur optischen Erkennung von Flüssigkeiten in dem Hohlkörper bzw. Schlauch vorgesehen ist **dadurch gekennzeichnet daß** die Lichtstrahlen reflektierende Einrichtung als Prisma (4, 4') ausgebildet ist.

12. Hohlkörper nach Anspruch 11, wobei die Lichtstrahlen brechende bzw reflektierende Einrichtung nach einem der Ansprüche 2 bis 8 ausgebildet ist.

## Claims

1. Spinal cannula having a needle hub (2), which has a transparent area for observing the liquor flowing through a hollow cavity (3) of the needle hub, wherein a means for refracting or reflecting rays of light is provided in or at the transparent area of the needle hub (2),
**characterised in that**
the means for reflecting rays of light is formed as a prism (4, 4').

2. Spinal cannula according to claim 1, wherein the prism (4') is mounted on the outer circumference of the needle hub (2) or of the transparent area.

3. Spinal cannula according to claim 2, wherein rows of outer prisms (40) are formed along the circumference offset to each other in the axial direction, and the prisms in each row are at a distance from each other in the circumferential direction.

4. Spinal cannula according to claim 2 or 3, wherein seen in the viewing direction (X) the outer prism (4') is associated with at least one bent area of the hollow cavity (3) or of the outer circumference of the needle hub (2) as the means for refracting rays of light.

5. Spinal cannula according to claim 1, wherein the prism (4) is formed on the inner circumference of an observation chamber (3) in the needle hub (2).

6. Spinal cannula according to claim 5, wherein a plurality of inner prisms (4) are formed adjacent each other on the inner circumference of the observation chamber (3) in the needle hub (2).

7. Spinal cannula according to one of claims 1 to 6, wherein at least one prism area delimits the hollow cavity or the observation chamber (3) in the needle hub (2).

8. Spinal cannula according to one of claims 1 to 7, wherein the prism (4, 4') or the prisms is or are moulded on to the spinal cannula.

9. Spinal cannula, having a needle hub (2), which has a transparent area for observing the liquor flowing through a hollow cavity (3) of the needle hub, wherein a means for refracting or reflecting rays of light is provided in or at the transparent area of the needle hub (2), **characterised in that** the means for reflecting rays of light is formed as a reflecting area (10) which is applied on the outer circumference in the transparent area of the needle hub wherein the reflecting side faces the hollow cavity (3).

10. Spinal cannula according to one of the preceding claims, wherein the spinal cannula is surrounded by a grip collar (8) having at least one recess (8a) on the circumference which is arranged over the means for refracting or reflecting rays of light.

11. Hollow body, especially a flexible tube having at least one transparent wall portion, wherein a means (4, 4', 10) for refracting or reflecting rays of light is provided in the area of the transparent wall portion for optical recognition of fluids in the hollow body or flexible tube, **characterised in that** the means for reflecting rays of light is formed as a prism (4, 4').

12. Hollow body according to claim 11, wherein the means for refracting or reflecting rays of light is formed according to one of claims 2 to 8.

## Revendications

1. Canule spinale munie d'un embout de canule (2) qui présente une zone transparente pour l'observation du liquide céphalorachidien s'écoulant par un espace creux (3) de l'embout de canule, un dispositif réfléchissant ou réfractant des rayons lumineux étant prévu dans ou sur la zone transparente de l'embout de canule (2), **caractérisée en ce que**, le dispositif réfléchissant des rayons lumineux est réalisé comme un prisme (4, 4').

2. Canule spinale selon la revendication 1, dans laquelle le prisme (4') est monté sur la périphérie extérieure de l'embout de canule (2) ou de la zone transparente.

3. Canule spinale selon la revendication 2, dans laquelle des rangées de prismes extérieurs (40) sont réalisées le long de la périphérie en déport dans le sens axial les unes par rapport aux autres et les prismes présentant un écartement dans chaque rangée dans le sens périphérique.

4. Canule spinale selon la revendication 2 ou 3, dans laquelle au moins une surface courbée de l'espace creux (3) ou de la périphérie extérieure de l'embout de canule (2) est associée au prisme extérieur (4') dans le sens d'observation (X) comme dispositif réfractant des rayons lumineux.

5. Canule spinale selon la revendication 1, dans laquelle le prisme (4) est réalisé sur la périphérie intérieure d'une chambre d'observation (3) dans l'embout de canule (2).

6. Canule spinale selon la revendication 5, dans laquelle plusieurs prismes intérieurs (4) sont réalisés les uns à côté des autres sur la périphérie intérieure de la chambre d'observation (3) dans l'embout de canule (2).

7. Canule spinale selon l'une quelconque des revendications 1 à 6, dans laquelle au moins une surface de prisme délimite l'espace creux ou la chambre d'observation (3) dans l'embout de canule (2).

8. Canule spinale selon l'une quelconque des revendications 1 à 7, dans laquelle le prisme (4, 4') ou les prismes sont formés sur la canule spinale.

9. Canule spinale munie d'un embout de canule (2) qui présente une zone transparente pour l'observation du liquide céphalorachidien s'écoulant par un espace creux (3) de l'embout de canule, un dispositif réfléchissant ou réfractant des rayons lumineux étant prévu dans ou sur la zone transparente de l'embout de canule (2), **caractérisée en ce que** le dispositif réfléchissant des rayons lumineux est réalisé comme une surface miroitante (10) appliquée sur la périphérie extérieure dans la zone transparente de l'embout de canule, le côté miroitant étant tourné vers l'espace creux (3).

10. Canule spinale selon l'une quelconque des revendications précédentes, dans laquelle la canule spinale est entourée sur la périphérie par un manchon de préhension (8) doté d'au moins un évidement (8a) qui est disposé au-dessus du dispositif réfléchissant ou réfractant des rayons lumineux.

11. Corps creux, en particulier tuyau flexible, muni d'au moins une section de paroi transparente, dans lequel un dispositif réfléchissant ou réfractant des rayons lumineux (4, 4', 10) est prévu dans la zone de la section de paroi transparente pour la détection optique de liquides dans le corps creux ou tuyau flexible, **caractérisé en ce que** le dispositif réfléchissant des rayons lumineux est réalisé comme un prisme (4, 4').

12. Corps creux selon la revendication 11, dans lequel le dispositif réfléchissant ou réfractant des rayons lumineux est réalisé selon l'une quelconque des revendications 2 à 8.
